# EUROPEAN PATENT SPECIFICATION

(11) **EP 3 484 565 B1**
(45) Date of publication and mention of the grant of the patent: **20.04.2022**
(21) Application number: 17743402.4
(22) Date of filing: 13.07.2017
(51) Int. Cl.: A61M 16/16, A61M 11/04, A61M 16/10

(54) **AN ELECTRICALLY POWERED HEATER UNIT**
ELEKTRISCH BETRIEBENE HEIZEREINHEIT
UNITÉ DE CHAUFFAGE À ALIMENTATION ÉLECTRIQUE

(30) Priority: 13.07.2016 IE 20160186
(43) Date of publication of application: 22.05.2019
(73) Proprietor: Oglesby & Butler Research & Development Limited, Carlow (IE)
(72) Inventor: STOREY, John, Joseph, Portlaoise County Laois (IE); GRIFFIN, Michael, John, Milford County Carlow (IE); TOWNSEND, John, Raheendoran County Carlow (IE)
(74) Representative: Gorman, Francis Fergus
(86) International application number: PCT/IE2017/000013
(87) International publication number: WO 2018/011778

(56) References cited:
- EP-A1- 0 591 537
- EP-A2- 0 290 159
- WO-A1-2006/046209
- WO-A1-2008/076455
- WO-A1-2009/002431
- WO-A2-2008/069907
- WO-A2-2015/056252
- US-A1- 2015 139 632

## Description

The present invention relates to an electrically powered heater unit, and the invention also relates to a method for producing an electrically powered heater unit.

Electrically powered heater units are known, and such electrically powered heater units are commonly used in evaporators, for example, evaporators for evaporating essential oils and the like. In general, such electrically powered heater units comprise an electrically resistive heating element, which may be located around or beneath a container forming a well for the essential oil to be evaporated. It is also known to provide electrically powered heating elements for heating tobacco and other herbal matter in a vaporising chamber in order to release volatiles in the herbal matter for inhaling thereof. Again, such heating elements tend to comprise an electrically resistive heating element. In general, the use of such electrically resistive heating elements tends to lead to a relatively cumbersome and somewhat inefficient heating unit due to the fact that the electrically resistive heating element cannot be brought into direct contact with an electrically conductive heat transfer element for transferring heat by conduction from the heating element to, for example, the container forming the well for the essential oil, or to a chamber containing the herbal matter. In general heat from such electrically resistive heating elements is transferred by radiation and convection. However, in general, it has been found that the efficiency with which heat can be transferred by convection and radiation is relatively poor. Furthermore, the control of the temperature at which the material to be heated, for example, essential oils, herbal matter and the like is difficult to control when heat is being transferred by radiation and/or convection, and in general, the precision with which the temperature of the material to be heated can be controlled is relatively low.

One such electrically powered heating unit is disclosed in PCT Specification No. WO 2015/056252 of Storey et al. The electrically powered heating unit of Storey comprises an electrically resistive heating element of serpentine shape. However, an insulating medium must be located between the electrically resistive heating element and any heat transfer element which is of an electrically conductive material. Since most good heat conductive materials which are suitable for heat transfer elements are electrically conductive, the requirement to provide an insulating medium between the electrical resistive heating element and the heat transfer element does to some extend reduce the efficiency of the heating unit.

PCT Specification No. WO 2008/076455 of Thuot discloses a positive temperature coefficient electrical heating device which is suitable for vaporising air treatment chemicals. The device of Thuot comprises a positive temperature coefficient heating element located in a housing between a resilient electrical contact element and a heat transfer electrical contact element. First and second electrical connectors are provided to the electrically conductive contact element and the electrically conductive heat transfer element, respectively, for connecting to an electricity supply.

PCT Specification No. WO 2006/046209 of Zobele discloses a positive temperature coefficient electrical heating device for vaporising a fragrance or an insecticide. The device of Zobele comprises a positive temperature coefficient heating element located between a resilient electrically conductive contact element and an electrically conductive heat transfer element which are housed together in a housing. First and second electrically conductive connector elements are provided to the electrical contact element and the heat transfer element, respectively, for connecting the device to an electrical power supply.

PCT Specification No. WO 2009/002431 of Adair discloses an electrical heating device for vaporising air treatment chemicals. The device comprises a housing within which is located a positive temperature coefficient heating element located between a resilient electrically conductive contact element and a heat diffuser plate. First and second electrical connecting elements are connected to the electrical contact element and the heat diffuser plate for connecting the device to an electrical power supply.

U.S. Patent Application Specification No. 2015/0139632 of Mueller et al. discloses a heating device for heating fluid passing through a pipeline. The heating device of Mueller comprises a housing having a fluid accommodating bore therethrough terminating at its opposite ends in couplers for coupling to a pipe through which the fluid to be heated flows. A copper tube is located in the bore of the pipe for transferring heat from the heating device to the fluid. A flattened portion of the copper tube extends into a U-shaped housing which is secured to the flattened portion of the copper tube. Located between the U-shaped housing and the flattened portion of the copper tube is a positive temperature coefficient disc heating element located between a pair of electrically conductive contacts for applying an electrical supply to the positive temperature coefficient heating element. A compression spring acting between the U-shaped housing and one of the contact elements urges the two contact elements with the heater element sandwiched therebetween into contact with the heater element, and in turn urges the other one of the contact elements into heat transfer engagement with the flattened portion of the copper tube for transferring heat from the heating element into the copper tube.

There is therefore a need for an electrically powered heater unit which provides improved efficiency in the transfer of heat from the heating element to the material to be heated by the heating unit, and in particular, to improve the efficiency of heat transfer by conduction from the heating element to the material to be heated.

The present invention is directed towards providing such an electrically powered heater unit, and the invention is also directed towards a method for producing such an electrically powered heating element.

According to the invention there is provided an electrically powered heater unit comprising a support element, an electrically conductive heat transfer element, an electrically conductive contact element, a positive temperature coefficient disc heater element located between the heat transfer element and the contact element, a securing means securing the heater element and the contact element between the heat transfer element and the support element and securing the heater element in electrical contact with the contact element and the heat transfer element, a first connecting means co-operating with the contact element for electrically connecting the contact element with a first pole of an electrical power supply, and a second connecting means co-operating with the heat transfer element for connecting the heat transfer element to a second pole of the electrical power supply, wherein an urging means comprising a compression spring cooperable with the support element and the contact element urges the contact element and the heat transfer element into the electrical contact with the heater element, and the securing means is configured to secure the heater element sandwiched tightly between the contact element and the heat transfer element, wherein the first connecting means extends centrally from the contact element, centrally through the compression spring, and centrally through the support element.

In one aspect of the invention the heater element defines opposite first and second planar major abutment surfaces, the first abutment surface being configured for abutting the contact element, and the second abutment surface of the heater element being configured for abutting the heat transfer element. Preferably, the first and second abutment surfaces of the heater element are parallel to each other.

In another aspect of the invention the first abutment surface of the heater element engages the first surface of the contact element with electrical conducting engagement. Preferably, the second abutment surface of the heater element engages the heat transfer element with electrical conducting engagement.

Advantageously, the second abutment surface of the heater element engages the heat transfer element with heat conducting engagement.

In another aspect of the invention the contact element defines a first major planar surface, the first surface of the contact element being in electrical conducting engagement with the heater element. Preferably, the contact element defines a second major surface opposite the first surface thereof, and parallel thereto. Advantageously, the contact element comprises a disc member.

In one aspect of the invention the first connecting means is configured for one of spot welding to and crimping onto a first electrically conductive wire for connecting the contact element to the first pole of the electrical power supply. Preferably, the first connecting means defines an opening for accommodating the first wire of the electrical power supply therein. Preferably, the first connecting means is in electrical conductive engagement with the contact element.

In another aspect of the invention the first connecting means comprises a first connecting element extending from the contact element. Preferably, the first connecting element comprises a connecting tab extending from the contact element. Advantageously, the connecting tab is integrally formed with the contact element from the same piece of material as that from which the contact element is formed.

In one aspect of the invention the connecting tab extends from a peripheral edge of the contact element. Preferably, the connecting tab is bent at a first bend adjacent the peripheral edge of the contact element to form a first portion extending inwardly from the peripheral edge, and substantially parallel to the contact element. Advantageously, the connecting tab is bent at a second bend at a location spaced apart from the first bend defining a portion extending from the contact element. Ideally, the portion of the connecting tab extending from the second bend and from the contact element is bent at a third bend defining an intermediate portion extending between the second and third bend and a distal portion extending from the third bend, the distal portion being bent through an angle of approximately 180° adjacent the third bend to lie substantially parallel with the intermediate portion of the connecting tab, the distal portion of the connecting tab extending from the third bend towards the contact element. Preferably, the distal portion and the intermediate portion of the connecting tab are spaced apart from each other adjacent the third bend for defining the opening for accommodating the first wire of the electrical power supply therein.

In one aspect of the invention the second connecting means extends from the heat transfer element. Preferably, the second connecting means is in electrically conductive engagement with the heat transfer element.

In another aspect of the invention the heat transfer element comprises a contact area for electrically engaging the second abutment surface of the heater element. Preferably, the contact area of the heat transfer element defines a planar contact surface for engaging the second abutment surface of the heater element with electrical and heat conductive engagement. Advantageously, the contact area of the heat transfer element is located in a well formed in the heat transfer element.

In another aspect of the invention the heat transfer element comprises an outer peripheral portion extending around the contact area thereof. Preferably, the second connecting means extends from the outer peripheral portion of the heat transfer element.

In another aspect of the invention the second connecting means extends parallel to and spaced apart from the first connecting means.

Preferably, the second connecting means is located on the heat transfer element spaced apart from the heater element and the contact element and to one side thereof.

In one aspect of the invention the second connecting means extends through the support element.

In one aspect of the invention the second connecting means is configured for one of soldering to and crimping onto a second electrically conductive wire of the electrical power supply for coupling the heat transfer element to the second pole of the electrical power supply.

In another aspect of the invention the second connecting means comprises a second connecting element secured to the heat transfer element. Preferably, the second connecting element is secured to the heat transfer element by being a press fit in a bore extending through the heat transfer element.

In another aspect of the invention the second connecting element defines a wire accommodating bore adjacent a distal end thereof for accommodating the second electrically conductive wire of the electrical power supply. Advantageously, the distal end of the second connecting element is configured for one of soldering or crimping the distal end of the connecting element to the second electrically conductive wire.

In one aspect of the invention the heat transfer element defines an outer heat transfer surface configured for transferring heat therefrom. Preferably, the outer heat transfer surface of the heat transfer element is configured for transferring heat therefrom by heat conduction. Advantageously, the outer heat transfer surface of the heat transfer element comprises a planar surface. Advantageously, the outer heat transfer surface of the heat transfer element extends parallel to the contact surface of the heat transfer element. Preferably, the heater element is of circular shape.

Advantageously, the contact element is of circular shape.

Ideally, the contact surface of the heat transfer element is of circular shape.

In another aspect of the invention the support element comprises a housing defining a hollow interior region therein for accommodating the heater element therein, the housing defining a communicating opening communicating with the hollow interior region, and the heat transfer element is located adjacent the communicating opening closing the communicating opening. Preferably, the housing comprises a plate member extending aground the housing adjacent the communicating opening. Advantageously, the heat transfer element is located abutting an outer surface of one of the housing and the plate member.

In another aspect of the invention the securing means comprises at least one pair of inter-engageable complementary formations, one of the formations of each pair thereof being provided on the heat transfer element and the other one of the pair of inter-engageable complementary formations being provided on the support element. Preferably, one of the inter-engageable complementary formations of each pair thereof comprises a retaining member extending from one of the support element and heat transfer element, and the other one of the inter-engageable complementary formations of each pair thereof comprising a corresponding receiver located in the other one of the support element and the heat transfer element. Advantageously, each retaining member comprises an elongated retaining member extending from the one of the support element and the heat transfer element. Ideally, an engagement ridge extends sidewardly from each retaining member for engaging the corresponding receiver in the other one of the pair of inter-engageable complementary formations of the support element and the heat transfer element. Preferably, the engagement ridge of each retaining member is shaped to pass through the communicating opening defined by the rim, the corresponding portion of which forms the receiver in one direction, and to engage the rim when urged in the opposite direction. Advantageously, the engagement ridge of each retaining member extends in a generally outwardly direction from the retaining member.

In one aspect of the invention the retaining member extends from the heat transfer element.

In another aspect of the invention the receiver of each pair of inter-engageable complementary formations comprises a portion of a rim defining the communicating opening in the support element.

Preferably, the inter-engageable complementary formations of each pair of inter-engageable complementary formations engage the other of the pair of inter-engageable complementary formations with a snap fit action.

In one aspect of the invention at least three pairs of inter-engageable complementary formations are provided spaced apart around the heater element. Preferably, six of the pairs of inter-engageable complementary formations are provided spaced apart around the heater element. Advantageously, the pairs of inter-engageable complementary formations are equi-spaced apart around the heater element.

Additionally, the invention provides a method for producing an electrically powered heater unit, the method comprising locating a positive temperature coefficient disc heater element between an electrically conductive heat transfer element and an electrically conductive contact element, securing the heater element and the contact element between the heat transfer element and a support element with the heater element in electrical contact with the contact element and the heat transfer element, providing a first connecting means co-operating with the contact element for electrically connecting the contact element with a first pole of an electrical power supply, and providing a second connecting means co-operating with the heat transfer element for connecting the heat transfer element to a second pole of the electrical power supply, wherein the method further comprises urging the contact element and the heat transfer element into electrical contact with the heater element by a compression spring cooperable with the support element and the contact element, and securing the heater element sandwiched tightly between the contact element and the heat transfer element, wherein the first connecting element is provided extending centrally from the contact element, centrally through the compression spring, and centrally through the support element. by

The advantages of the invention are many. A particularly important advantage of the invention is that it provides improved efficiency in the transfer of heat by conduction from an electrically powered heater element to the material to be heated by the heater unit. Because of the improved efficiency of heat transfer by conduction, the heater element is much more reactive to the temperature of the material or the container to be heated, and is considerably more reactive to the temperature of the heat transfer element through which heat is being transferred from the heater element to the material or the container for the material to be heated. Furthermore, the reaction time by the heater element to a change in temperature in the transfer element is almost instantaneous, since the heat transfer element is in direct heat conductive engagement with the heater element.

A further advantage of the invention is that by virtue of the fact that the contact element and the heat transfer element are both in direct electrically conductive engagement with the heater element, the construction of the heater unit is considerably simplified. This is due in particular, to the fact that the first connecting means for connecting the electrical power supply to the heater element can be directly connected to the contact element, and the second connecting means for connecting the electrical power supply to the heater element can be connected directly to the heat transfer element.

The invention will be more clearly understood from the following description of a preferred embodiment thereof which is given by way of example only with reference to the accompanying drawings, in which:

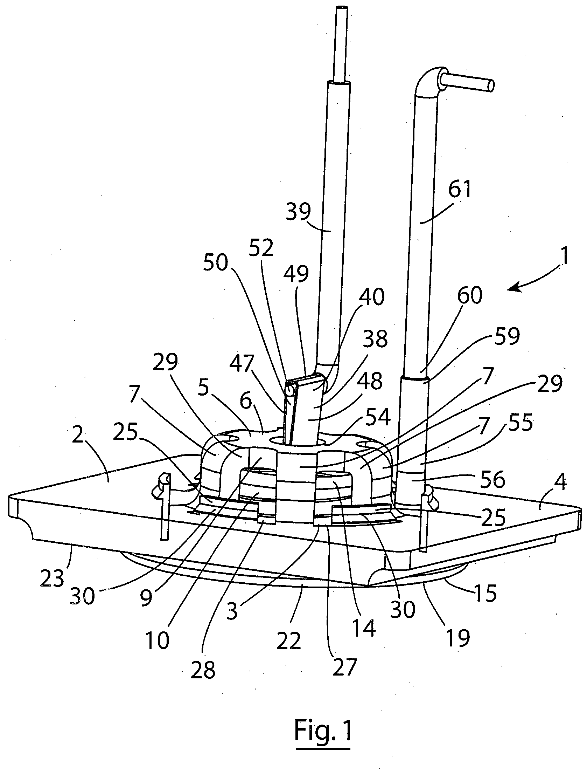

Fig. 1 is a perspective view of an electrically powered heater unit according to the invention,

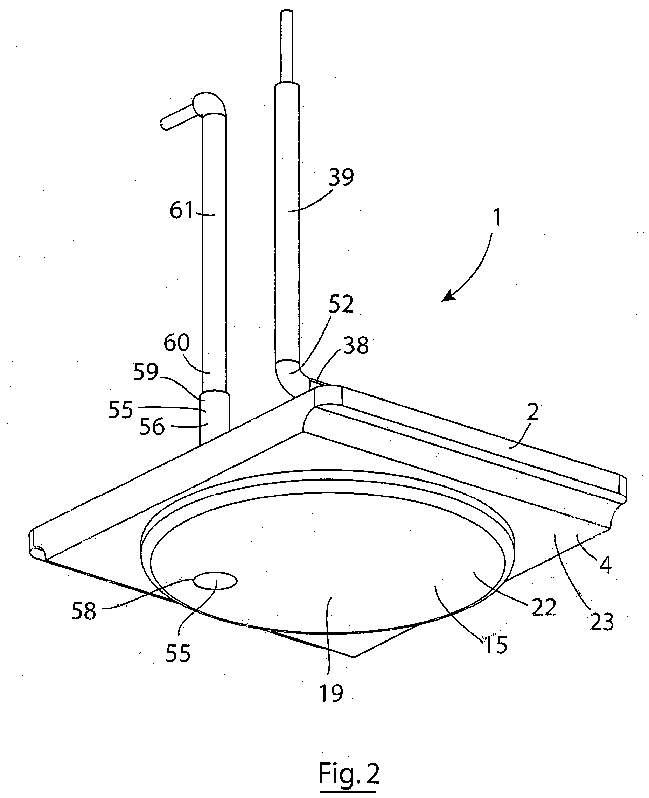

Fig. 2 is another perspective view of the heater unit of Fig. 1,

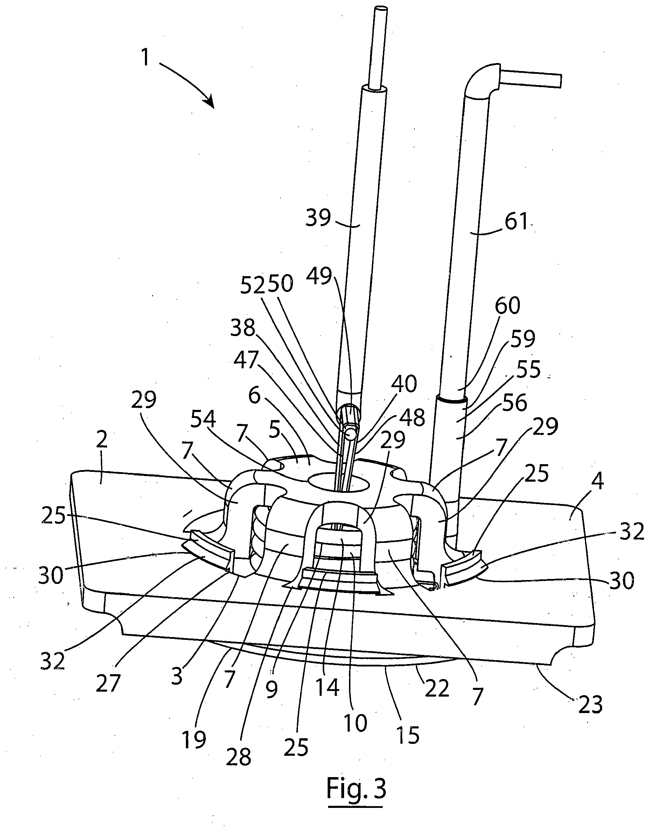

Fig. 3 is a further perspective view of the heater unit of Fig. 1,

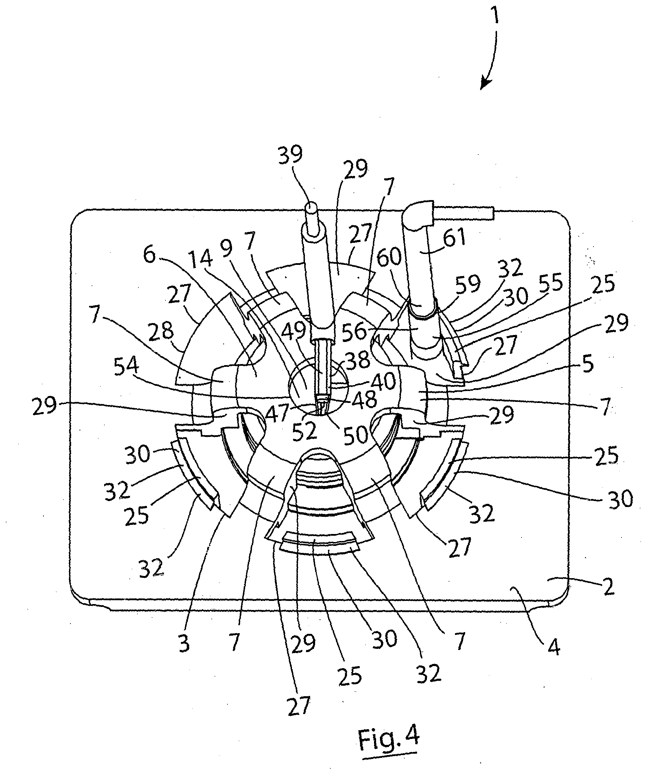

Fig. 4 is a still further perspective view of the heater unit of Fig. 1,

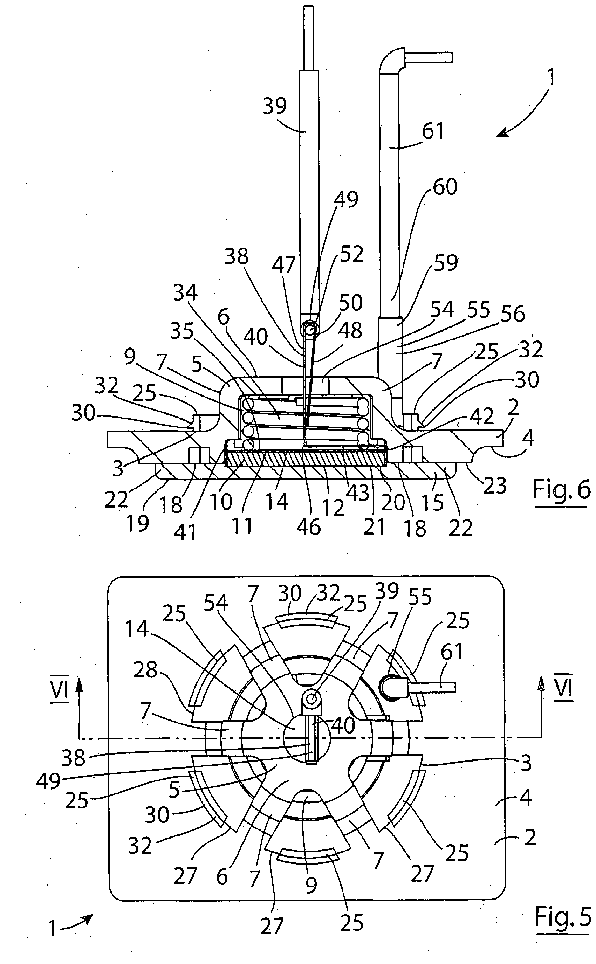

Fig. 5 is a top plan view of the heater unit of Fig. 1,
Fig. 6 is a cross-sectional front end elevational view of the heater unit of Fig. 1 on the line VI-VI of Fig. 5,

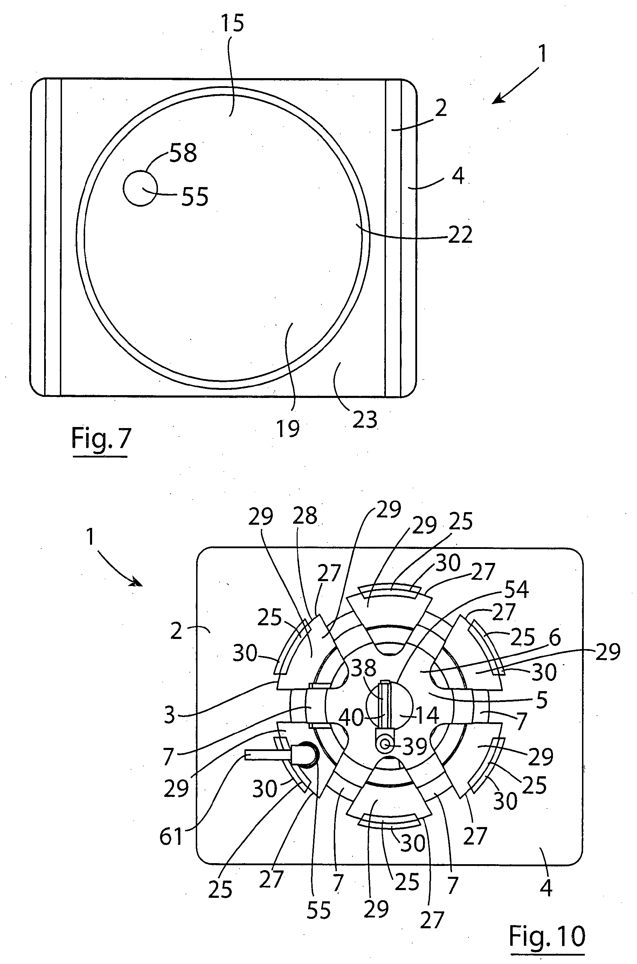

Fig. 7 is an underneath plan view of the heater unit of Fig. 1,

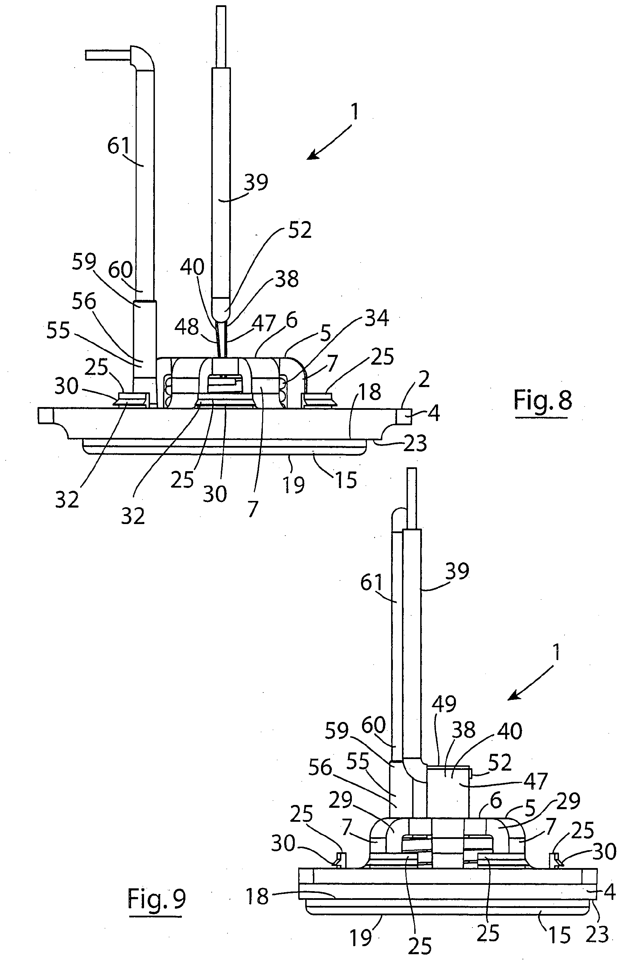

Fig. 8 is a rear end elevational view of the heater unit of Fig. 1,
Fig. 9 is a side elevational view of the heater unit of Fig. 1,
Fig. 10 is another top plan view of the heater unit of Fig. 1, and

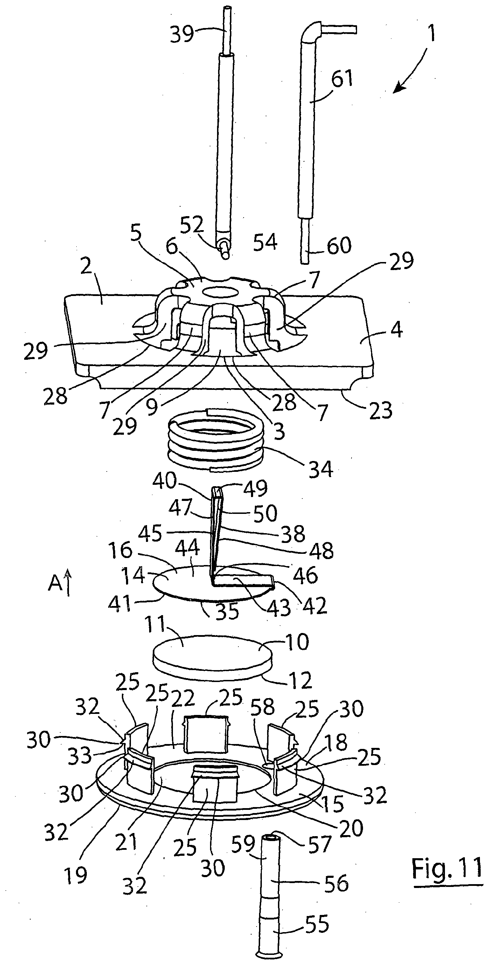

Fig. 11 is an exploded perspective view of the heater unit of Fig. 1.

Referring to the drawings there is illustrated an electrically powered heater unit according to the invention indicated generally by the reference numeral 1 which is suitable for heating many appliances, for example, an evaporator well of an evaporator for evaporating essential oils, a herb chamber for heating herbs in order to release volatiles therefrom, for example, for heating tobacco, but not burning the tobacco, for releasing volatiles from the tobacco, and for many other uses. Neither the evaporator well nor the evaporator, nor the herb chamber are illustrated, however such evaporator wells, evaporators, herb chambers of vaporisers will be well known to those skilled in the art.

The heater unit 1 comprises a support element 2 of a plastics material, which typically is formed by injection moulding, and comprises a plate member 4 having a central circular communicating opening 3 extending therethrough and a housing 5 centrally formed on the plate member 4. The housing 5 comprises a top wall 6 joined to the plate member 4 by a plurality of circumferentially spaced apart connecting members 7 extending radially outwardly and downwardly from the top wall 6 to the plate member 4 around and adjacent the communicating opening 3. The top wall 6 and the connecting members 7 together define a hollow interior region 9 of the housing 5. The communicating opening 3 in the plate member 4 communicates with and provides access into the hollow interior region 9.

A positive temperature coefficient disc heater element 10 of circular shape having a pair of opposite parallel first and second major planar abutment surfaces 11 and 12, respectively, is located in the hollow interior region 9. The heater element 10 is sandwiched between an electrically conductive contact element 14 which is located in the hollow interior region 9 and an electrically conductive heat transfer element 15. The heat transfer element 15 is secured to the plate member 4 in a manner which will be described below closing the communicating opening 3. The contact element 14 is provided in the form of a circular disc 16 of electrically conductive material, which in this case is stainless steel, and is configured as will be described below for applying a first pole of an electrical power supply to the adjacent first abutment surface 11 of the heater element 10.

The heat transfer element 15 is of circular shape and of an electrically conductive and heat conductive material, which in this case comprises nickel and iron plated brass. The brass of the heat transfer element 15 is initially iron plated to make it magnetically attractable, and is then nickel plated to prevent rusting of the iron plating. The heat transfer element 15 defines a planar inner surface 18, and a planar outer heat transfer surface 19. A central well 20 of circular shape and of diameter substantially similar to the diameter of the heater element 10 is formed centrally in the inner surface 18 of the heat transfer element 15 for centrally locating the heater element 10 in the heat transfer element 15. The central well 20 defines a central contact area, namely, a planar electrical contact surface 21 for engaging and abutting the adjacent second abutment surface 12 of the heater element 10 with electrical conductive engagement for applying a second pole of the electrical power supply to the second abutment surface 12 of the heater element 10. The contact surface 21 also engages the second abutment surface 12 of the heater element 10 with heat conductive engagement for transferring heat from the heater element 10 by heat conduction. The diameter of the heat transfer element 15 is greater than the diameter of the communicating opening 3 in the plate member 4, so that an outer peripheral portion 22 of the heat transfer element 15 extending around the central well 20 abuts an outer surface 23 of the plate member 4 adjacent the communicating opening 3. The inner surface 18, the outer surface 19 and the contact surface 21 of the heat transfer element 15 are parallel to each other.

A securing means for securing the heat transfer element 15 in tight abutting engagement with the plate member 4 comprises a plurality, in this embodiment of the invention six pairs of inter-engageable complementary formations, one of which inter-engageable complementary formations of each pair thereof comprises a retaining member 25 extending from the inner surface 18 of the heat transfer element 15 adjacent the peripheral area 22 thereof. The other one of the inter-engageable complementary formations of each pair thereof comprises a corresponding receiver provided by a receiving portion 27 of the rim 28 defining the communicating opening 3 in the plate member 4. Openings 29 defined between adjacent pairs of the connecting members 7 of the housing 5 accommodate the retaining members 25.

Each retaining member 25 comprises a corresponding engagement ridge 30 which extends outwardly from the corresponding retaining member 25 for engaging the corresponding receiving portion 27 of the rim 28 with a snap-fit action. Each engagement ridge 30 defines an angled lead-in surface 32 to permit passage of the corresponding retaining member 25 in the direction of the arrow A through the communicating opening 3, and a trailing surface 33 extending substantially perpendicularly or at an acute angle to the corresponding retaining member 25 for engaging the corresponding receiving portion 27 of the rim 28 of the communicating opening 3 in order that the co-operating action between the retaining members 25, the engagement ridges 30 thereof and the retaining portions 27 of the rim 28 retains the heat transfer element 15 in tight abutting engagement with the plate member 4 with the inner surface 18 of the heat transfer element 15 in tight secure abutting engagement with the outer surface 23 of the plate member 4.

An urging means comprising a compression spring 34 is located in the hollow interior region 9 of the housing 5 and acts between the top wall 6 of the housing 5 and the contact element 14 for urging a first major planar surface 35 of the contact element 14 into tight electrical conducting abutting engagement with the first abutment surface 11 of the heater element 10, and in turn for urging the contact surface 21 of the heat transfer element 15 into tight electrical conducing engagement with the second abutment surface 12 of the heater element 10, so that an electrical power supply applied across the contact element 14 and the heat transfer element 15 is correspondingly applied across the heater element 10 through the respective first and second abutment surfaces 11 and 12 of the heater element 10.

A first connecting means for connecting the first pole of the electrical power supply to the contact element 14, in this embodiment of the invention comprises a first connecting element 38 extending from the contact element 14 for spot welding to a first electrically conductive wire 39 of the electrical power supply. The first connecting element 38 comprises a connecting tab 40 which is integrally formed with the contact disc 16 from the same piece of material as that from which the contact disc 16 is formed. The connecting tag 40 extends from a peripheral edge 41 of the contact disc 16. The connecting tab 40 is bent at a first bend 42 adjacent the peripheral edge 41 to form a first portion 43 which extends inwardly from the peripheral edge 41 and lies parallel with and adjacent a second major planar surface 44 of the contact disc 16. The second surface 44 of the contact element 14 is opposite and parallel to the first surface 35 thereof which tightly abuts and engages the adjacent first abutment surface 11 of the heater element 10.

The connecting tab 40 is then bent at a second bend 46 adjacent the centre of the contact disc 16, and a portion 45 of the connecting tab 40 extending from the second bend 46 extends perpendicularly from the contact disc 16. The portion 45 of the connecting tab 40 extending from the second bend 46 is bent at a third bend 49 spaced apart from the second bend 46 to form the portion 45 of the connecting tab 40 into an intermediate portion 47 and a distal portion 48. The distal portion 48 of the tab 40 is bent at the third bend 49 through approximately 180° relative to the intermediate portion 47 to extend back towards the contact disc 16 substantially adjacent the intermediate portion 47 of the connecting tab 40. The distal portion 48 is slightly spaced apart from the intermediate portion 47 of the connecting tab 40 adjacent the third bend 49 to define an opening 50 for accommodating a proximal portion 52 of the first wire 39, so that the distal portion 48 and the intermediate portion 47 of the connecting tab 40 can be spot welded to the first wire 39 or crimped onto the first wire 39 in order that the connecting tab 40 is secured to the first wire 39 with good electrical continuity. The intermediate portion 47 and the distal portion 48 of the connecting tab 40 extend centrally through the compression spring 34.

A central opening 54 in the top wall 6 of the housing 5 accommodates the distal portion 48 and the intermediate portion 47 of the connecting tab 40 centrally through the housing 5 from the hollow interior region 9 for engaging the proximal portion 52 of the first wire 39 externally of the housing 5.

A second connecting means for connecting the second pole of the electrical power supply to the heat transfer element 15, in this embodiment of the invention comprises a second connecting element 55 formed by a tubular element 56. The tubular element 56 is engaged in a bore 58 extending through the heat transfer element 15 in the outer peripheral portion 22 thereof. The tubular element 56 and the bore 58 are dimensioned so that the tubular element 56 is a tight press fit in the bore 58 thus making a secure electrically conductive joint between the tubular element 56 and the heat transfer element 15. The tubular element 56 extends through one of the openings 29 defined between two adjacent ones of the connecting members 7 of the housing 5 of the support element 2. A bore 57 extends into the tubular element 56 from the distal end 59 thereof for receiving a proximal end 60 of a second wire 61 of the electrical power supply. The diameter of the bore 57 is such as to accommodate the proximal end 60 of the second wire 61 therein so that the distal end 59 of the tubular element 56 can be crimped onto the proximal end 60 of the second wire 61 or the second wire 61 can be soldered to the tubular element 56 in the bore 57.

In this embodiment of the invention the heater element 10 is suitable for powering by a 3.7 volt DC electrical power supply, and is suitable for battery powering. The heater element 10 in this embodiment of the invention is configured to operate within a temperature in the range of 80°C to 103°C, which is a suitable temperature range for evaporating essential oils. Being a positive temperature coefficient heating element, the temperature of the heating element 10 rises rapidly to 80°C, and on reaching the temperature of 80°C, the current drawn by the heater element 10 falls off rapidly so that the temperature then rises relatively slowly to the maximum temperature of 103°C. When the heater element 10 is selected such that the heat output of the heater element 10 is matched with the heat requirement of the load, the temperature of the heater element 10, in general, stabilises at a working temperature in the order of 103°C. As further heat is drawn from the heat transfer element 15, the temperature of the heater element 10 commences to fall. As the temperature of the heater element 10 falls, the current drawn by the heater element 10 progressively increases, thereby relatively rapidly returning the temperature of the heater element 10 to its working temperature of the order of 103°C. As less heat is drawn from the heat transfer element 15 and the temperature of the heater element 10 commences to rise, the current drawn by the heater element 10 progressively decreases, thereby the temperature of the heater element 10 is maintained substantially constant around the working temperature of the order of 103°C.

The heater element 10 is of thickness t of approximately 0.9mm between the opposite first and second abutment surfaces 11 and 12 thereof. The central well 20 in the heat transfer element 15 is relatively shallow, and in this embodiment of the invention is of depth not more than 0.2mm. Thus, the heat transfer element 15 contacts the second abutment surface 12 of the heater element 10, and the contact element 14 contacts the first abutment surface 11 of the heater element 10, so that the voltage of the electrical power supply which is applied to the heater element 10 is applied to the respective opposite first and second abutment surfaces 11 and 12 of the heater element 10 by the contact element 14 and the heat transfer element 15, respectively.

In use, the heater unit 1 is assembled by initially forming the first connecting element 38 in the form of the connecting tab 40 of the contact element 14. The second connecting element 55 is secured in the bore 58 of the heat transfer element 15 by being a press fit therein. The heater element 10 is then placed in the central well 20 of the heat transfer element 15 with the second abutment surface 12 of the heater element 10 abutting the contact surface 21 of the heat transfer element 15. The contact element 14 is placed on top of the heater element 10 with the first surface 35 of the contact element 14 abutting the first abutment surface 11 of the heater element 10, and with the first connecting element 38 facing away from the heater element 10. The compression spring 34 is then placed on top of the contact element 14 abutting the second surface 44 thereof, with the first connecting element 38 extending centrally through the compression spring 34.

The support element 2 is then brought downwardly over the heat transfer element 15 with the outer surface 23 of the plate member 4 facing the inner surface 18 of the heat transfer element 15 and with the well 20, the heater element 10, the contact element 14 and the connecting tab 40 centrally aligned with the housing 5. As the support element 2 is being brought downwardly over the heat transfer element 15, the second connecting element 55 is aligned with one of the openings 29 between an adjacent pair of the connecting members 7, and the retaining members 25 are aligned with corresponding ones of the openings 29. As the support element 2 is brought further downwardly over the heat transfer element 15 the first connecting element 38 extends through the central opening 54 in the top wall 6 of the housing 5, and the second connecting element 55 extends through the opening 29 with which it is aligned. The retaining members 25 then pass through the communicating opening 3 in the plate member 4, and into the corresponding openings 29. As the engagement ridges 30 pass the corresponding receiving portions 27 of the rim 28 defining the communicating opening 3, the engagement ridges 30 snap into engagement with the receiving portions 27 of the rim 28 with a snap-fit action, thereby retaining the inner surface 18 of the peripheral portion 22 of the heat transfer element 15 in tight abutting engagement with the outer surface 23 of the plate member 4, with the heater element 10, the contact element 14 and the compression spring 34 located in the hollow interior region 9 of the housing 5.

With the heat transfer element 15 secured to the support element 2, the action of the compression spring 34 between the top wall 6 of the housing 5 and the contact element 14 ensures tight abutting electrically conductive engagement between the first surface 35 of the contact element 14 and the first abutment surface 11 of the heater element 10, and between the contact surface 21 in the central well 20 of the heat transfer element 15 and the second abutment surface 12 of the heater element 10. The tight abutting engagement between the second abutment surface 12 of the heater element 10 and the contact surface 21 of the heat transfer element 15 also ensures good heat conductive engagement between the second abutment surface 12 of the heater element 10 and the contact surface 21 of the heat transfer element 15, thereby ensuring efficient heat transfer from the heater element 10 to the heat transfer element 15 by heat conduction.

The first connecting element 38 is then spot welded or crimped onto the first wire 39, and the second wire 61 is secured in the bore 57 of the second connecting element 55 by crimping or by soldering. The heater element 1 is then ready for use.

By virtue of the fact that the heat transfer element 15 is of nickel and iron plated brass, the heat transfer element is of a heat conductive material, and by virtue of the fact that the heater element 10 is in tight abutting engagement with the contact surface 21 in the central well 20 of the heat transfer element 15, heat is efficiently transferred from the heater element 10 to the heat transfer element 15 by heat conduction. Thus, the heat transfer element 15 can be used as a hot plate for many heating products and appliances and in particular, battery powered portable heating products and appliances, for example, for heating a container defining a well for essential oil of an essential oil evaporator, for heating herb material, for example, tobacco in a herb chamber of a vaporiser for releasing volatiles of the tobacco or other herb. The herb chamber could be constructed with the heat transfer element 15 forming a base of, for example, a cylindrical chamber or other shaped chamber for holding the herbal matter to be heated. Such a vaporiser would be of the "heat not burn" type of vaporiser for heating but not burning tobacco or other herbal matter for releasing the volatiles therein for inhaling by a subject. The heat transfer element 15 could also be used as a hot plate for a cooker, for maintaining a cooking utensil warm or indeed for cooking food in a cooking utensil. The heat transfer element 15 could also be used as a hot plate of a chafing heater for use in a chafing trolley for maintaining food warm.

Additionally, by virtue of the fact that the heat transfer element 15 includes iron plating, the heat transfer element is magnetically attractable, and thus, a container defining a well for holding an essential oil to be evaporated could be releaseably secured to the heat transfer element 15 by a magnet, which could be secured to or embedded in the base or in any other suitable location in the container defining the essential oil containing well. Alternatively, the heat transfer element 15 as well as being of a heat conductive material and an electrically conductive material may also be configured as a permanent magnet.

While the support element has been described as being of a plastics material injection moulded, it will of course be appreciated that it will be of a heat resistant plastics material. Needless to say, the support element may be of any other suitable material.

It will also be appreciated that while the support element has been described as comprising a plate member with the housing 5 integrally formed with the plate member, the support element may be of any other suitable construction, and in some embodiments of the invention it is envisaged that the plate member may be dispensed with, and the connecting members would terminate in a ring the inner rim of which would define the communicating opening to the hollow interior region of the housing, and the receiving portions 27 for engaging the retaining members 25. The outer peripheral portion of the heat transfer element would then abut the ring.

It will also be appreciated that while the housing has been described as being a housing, the housing when located on a plate member need not necessarily be centrally located on the plate member. Furthermore, while the housing has been described as comprising a top wall and connecting members extending downwardly from the top wall to the plate member, any other suitable construction of housing could be provided. Indeed, the housing could be formed from a top wall and a side wall extending around the top wall and terminating in the plate member or a ring as discussed above.

It will also be appreciated that the heat transfer element may be of any other suitable material besides nickel and iron plated brass. However, it is important that the heat transfer element be of both an electrically conductive material and a heat conductive material.

The contact element may also be of any other suitable electrical conductive material besides stainless steel.

While the heater element has been described as being configured to operate within a working temperature range of 80°C to 103°C, the working temperature range for which the heater element 10 will operate will be selected depending on the use to which the heater unit is being put. For example, for heating an essential oil evaporator well, it is envisaged that the working temperature range of the heater element will be in the order of 80°C to 103°C, while on the other hand, when the heater unit is for use in a vaporiser for vaporising herbal matter, it is envisaged that the working temperature range of the heater element will be within the range of 170'C to 230°C. Additionally, the working temperature range of the heater element will be dependent on the temperature to which the herbal matter is to be heated in order to release the volatiles which are to be released. In cases where the heater unit is adapted for other uses and purposes, the heater element will be selected to operate within a working temperature range appropriate to the use or purpose for which the heater unit is to serve.

While the heater element has been described as comprising a circular disc heater element, the heater element may be of any other shape besides circular, for example, the disc heater element may be of square, rectangular shape or any other polygonal shape. While it would not be necessary, it nonetheless is envisaged that the contact element and the shape of the well within which the contact surface is located in the heat transfer element would typically be of similar shape to that of the disc heater element.

While the securing means for securing the heat transfer element to the support element has been described as comprising a plurality of inter-engageable complementary formations, any other type of securing means, for example, other types of inter-engageable complementary formations could be used. Alternatively, the securing means could comprise a fastener or fasteners, for example, screws, rivets, or the like which would secure the heat transfer element to the support element.

## Claims

1. An electrically powered heater unit comprising a support element (2), an electrically conductive heat transfer element (15), an electrically conductive contact element (14), a positive temperature coefficient disc heater element (10) located between the heat transfer element (15) and the contact element (14), a securing means (25,27,28) securing the heater element (10) and the contact element (14) between the heat transfer element (15) and the support element (2) and securing the heater element (10) in electrical contact with the contact element (14) and the heat transfer element (15), a first connecting means (38) co-operating with the contact element (14) for electrically connecting the contact element (14) with a first pole of an electrical power supply, and a second connecting means (55) co-operating with the heat transfer element (15) for connecting the heat transfer element (15) to a second pole of the electrical power supply, wherein an urging means (34) comprising a compression spring (34) cooperable with the support element (2) and the contact element (14) urges the contact element (14) and the heat transfer element (15) into the electrical contact with the heater element (10), and the securing means (25,27,28) is configured to secure the heater element (10) sandwiched tightly between the contact element (14) and the heat transfer element (15), **characterised in that** the first connecting means (38) extends centrally from the contact element (14), centrally through the compression spring (34), and centrally through the support element (2).

2. An electrically powered heater unit as claimed in Claim 1 **characterised in that** the first connecting means (38) comprises a first connecting tab (40) extending from a peripheral edge (41) of the contact element (14), the connecting tab (40) being bent at a first bend (42) adjacent the peripheral edge (41) of the contact element (14) to form a first portion (43) extending inwardly from the peripheral edge (41), and substantially parallel to the contact element (14), the connecting tab (40) being bent at a second bend (46) at a location spaced apart from the first bend (42) and defining a portion (45) extending from the contact element (14).

3. An electrically powered heater unit as claimed in Claim 2 **characterised in that** the portion (45) of the connecting tab (40) extending from the second bend (46) and from the contact element (14) is bent at a third bend (49) defining an intermediate portion (47) extending between the second and third bends (46,49) and a distal portion (48) extending from the third bend (49), the distal portion (48) being bent through an angle of approximately 180° adjacent the third bend (49) to lie substantially parallel with the intermediate portion (47) of the connecting tab (40), the distal portion (48) of the connecting tab (40) extending from the third bend (49) towards the contact element (14), the distal portion (48) and the intermediate portion (47) of the connecting tab (40) are spaced apart from each other adjacent the third bend (49) for defining an opening (50) for accommodating a first electrically conductive wire (39) for connecting the contact element (14) to a first pole of the electrical power supply therein.

4. An electrically powered heater unit as claimed in any preceding claim **characterised in that** the second connecting means (55) is located on the heat transfer element (15) spaced apart from the heater element (10) and the contact element (14) and to one side thereof, and extends parallel to and spaced apart from the first connecting means (38), and through the support element (27).

5. An electrically powered heater unit as claimed in any preceding claim **characterised in that** the heater element (10) defines opposite first and second planar major abutment surfaces (11,12), the first abutment surface (11) of the heater element (10) being configured for abutting the contact element (14), and the second abutment surface (12) of the heater element (10) being configured for abutting the heat transfer element (15), the heat transfer element (15) comprising a contact area (21) defining a planar contact surface (21) for engaging the second abutment surface (12) of the heater element (10) with electrical and heat conductive engagement.

6. An electrically powered heater unit as claimed in Claim 5 **characterised in that** the heat transfer element (15) comprises an outer peripheral portion (22) extending around the contact area (21) thereof, and the second connecting means (55) extends from the outer peripheral portion (22) of the heat transfer element (15).

7. An electrically powered heater unit as claimed in any preceding claim **characterised in that** the second connecting means (55) comprises a second connecting element (55,56) secured to the heat transfer element (15), the second connecting element defining a wire accommodating bore (57) adjacent a distal end (59) thereof for accommodating a second electrically conductive wire (61) of a second pole of the electrical power supply, the distal end (59) of the second connecting element (55,56) being configured for one of soldering or crimping the distal end (59) of the connecting element (55,56) to the second electrically conductive wire.

8. An electrically powered heater unit as claimed in any preceding claim **characterised in that** the support element (2) comprises a housing (5) defining a hollow interior region (9) therein for accommodating the heater element (10) therein, the housing (5) defining a communicating opening (3) communicating with the hollow interior region (9), and comprising a plate member (4) extending around the housing (5) adjacent the communicating opening (3), and the heat transfer element (15) is located abutting an outer surface (23) of one of the housing (5) and the plate member (4) adjacent the communicating opening (3) and closing the communicating opening (3).

9. An electrically powered heater unit as claimed in Claim 8 **characterised in that** the securing means (25,27,28) comprises at least one pair of inter-engageable complementary formations (25,27,28), one of the formations (25) of each pair thereof being provided on the heat transfer element (15) and the other one of the pair of the inter-engageable complementary formations (25,27,28) being provided on the support element (2), one of the inter-engageable complementary formations (25,27,28) of each pair thereof comprising a retaining member (25) extending from one of the support element (2) and heat transfer element (15), and the other one of the pair of the inter-engageable complementary formations (25,27,28) of each pair thereof comprising a corresponding receiver (27,28) located in the other one of the support element (2) and the heat transfer element (15).

10. An electrically powered heater unit as claimed in Claim 9 **characterised in that** each retaining member (25) comprises an elongated retaining member (25) extending from the one of the support element (2) and the heat transfer element (15), and an engagement ridge (30) extends sidewardly from each retaining member (25) for engaging the corresponding receiver (27,28) of the other one of the pair of the inter-engageable complementary formations (25,27,28).

11. An electrically powered heater unit as claimed in Claim 10 **characterised in that** each retaining member (25) extends from the heat transfer element (15), the engagement ridge (30) of each retaining member (25) extends in a generally outwardly direction from the retaining member (25), and the receiver (27,28) of each pair of the inter-engageable complementary formations (25,27,28) comprises a portion (27) of a rim (28) defining the communicating opening (3) in the housing (5).

12. An electrically powered heater unit as claimed in Claim 11 **characterised in that** the engagement ridge (30) of each retaining member (25) is shaped to pass in one direction through the communicating opening (3) defined by the rim (28), the corresponding portion of which forms the receiver (27,28), and to engage the rim (28) when urged in the opposite direction.

13. A method for producing an electrically powered heater unit (1), the method comprising locating a positive temperature coefficient disc heater element (10) between an electrically conductive heat transfer element (15) and an electrically conductive contact element (14), securing the heater element (10) and the contact element (14) between the heat transfer element (15) and a support element (2) with the heater element (10) in electrical contact with the contact element (14) and the heat transfer element (15), providing a first connecting means (38) co-operating with the contact element (14) for electrically connecting the contact element (14) with a first pole of an electrical power supply, and providing a second connecting means (55) co-operating with the heat transfer element (15) for connecting the heat transfer element (15) to a second pole of the electrical power supply, wherein the method further comprises urging the contact element (14) and the heat transfer element (15) into electrical contact with the heater element (10) by a compression spring (34) cooperable with the support element (2) and the contact element (14), and securing the heater element (10) sandwiched tightly between the contact element (14) and the heat transfer element (15), **characterised in that** the first connecting element (38) is provided extending centrally from the contact element (14), centrally through the compression spring (34), and centrally through the support element (2).

## Patentansprüche

1. Elektrisch betriebene Heizeinheit mit einem Trägerelement (2), einem elektrisch leitenden Wärmeübertragungselement (15), einem elektrisch leitenden Kontaktelement (14), einem zwischen dem Wärmeübertragungselement (15) und dem Kontaktelement (14) angeordneten PTC-Scheibenheizelement (10), einer Befestigungseinrichtung (25, 27, 28), die das das Heizelement (10) und das Kontaktelement (14) zwischen dem Wärmeübertragungselement (15) und dem Trägerelement (2) befestigt und das Heizelement (10) in elektrischem Kontakt mit dem Kontaktelement (14) und dem Wärmeübertragungselement (15) hält, einer ersten Verbindungseinrichtung (38), die mit dem Kontaktelement (14) zusammenwirkt, um das Kontaktelement (14) mit einem ersten Pol einer elektrischen Stromversorgung zu verbinden, und einer zweiten Verbindungseinrichtung (55), die mit dem Wärmeübertragungselement (15) zusammenwirkt, um das Wärmeübertragungselement (15) mit einem zweiten Pol einer elektrischen Stromversorgung zu verbinden, wobei ein Druckmittel (34), das eine Druckfeder (34) umfasst, die mit dem Trägerelement (2) und dem Kontaktelement (14) zusammenwirken kann, das Kontaktelement (14) und das Wärmeübertragungselement (15) in elektrischen Kontakt mit dem Heizelement (10) drückt, und wobei die Befestigungseinrichtung (25, 27, 28) dazu ausgeführt ist, das Heizelement (10) fest zwischen dem Kontaktelement (14) und dem Wärmeübertragungselement (15) einzuschließen, **dadurch gekennzeichnet, dass** sich die erste Verbindungseinrichtung (38) mittig vom Kontaktelement (14), mittig durch die Druckfeder (34) und mittig durch das Trägerelement (2) erstreckt.

2. Elektrisch betriebene Heizeinheit nach Anspruch 1, **dadurch gekennzeichnet, dass** die erste Verbindungseinrichtung (38) eine erste Verbindungsfahne (40) aufweist, die sich von einem Umfangsrand (41) des Kontaktelements (14) aus erstreckt, wobei die Verbindungsfahne (40) an einer ersten Krümmung (42) neben dem Umfangsrand (41) des Kontaktelements (14) gebogen ist, um einen ersten Abschnitt (43) zu bilden, der von dem Umfangsrand (41) nach innen und im Wesentlichen parallel zum Kontaktelement (14) verläuft, wobei die Verbindungsfahne (40) an einer zweiten Krümmung (46) an einer von der ersten Krümmung (42) beabstandeten Stelle gebogen ist und einen Abschnitt (45) definiert, der sich von dem Kontaktelement (14) erstreckt.

3. Elektrisch betriebene Heizeinheit nach Anspruch 2, **dadurch gekennzeichnet, dass** der Abschnitt (45) der Verbindungsfahne (40), der sich von der zweiten Krümmung (46) und von dem Kontaktelement (14) erstreckt, an einer dritten Krümmung (49) gebogen ist, die einen Zwischenabschnitt (47) definiert, der zwischen der zweiten und dritten Krümmung (46, 49) verläuft und einen distalen Abschnitt (48) festlegt, der sich von der dritten Krümmung (49) aus erstreckt, wobei der distale Abschnitt (48) über einen Winkel von ungefähr 180° angrenzend an die dritte Krümmung (49) gebogen ist, um im Wesentlichen parallel zu dem mittleren Abschnitt (47) der Verbindungsfahne (40) zu liegen, wobei der distale Abschnitt (48) der Verbindungsfahne (40) sich von der dritten Krümmung (49) aus in Richtung des Kontaktelements (14) erstreckt, wobei der distale Abschnitt (48) und der Zwischenabschnitt (47) der Verbindungsfahne (40) nahe der dritten Krümmung (49) voneinander beabstandet sind, um eine Öffnung (50) zur Aufnahme eines ersten elektrisch leitenden Drahts (39) zur Verbindung des Kontaktelements (14) mit einem ersten Pol der elektrischen Stromversorgung darin zu bilden.

4. Elektrisch betriebene Heizeinheit nach einem der vorhergehenden Ansprüche, **dadurch gekennzeichnet, dass** die zweite Verbindungseinrichtung (55) auf dem Wärmeübertragungselement (15) im Abstand von dem Heizelement (10) und dem Kontaktelement (14) und auf einer Seite derselben angeordnet ist und parallel zu und im Abstand zu der ersten Verbindungseinrichtung (38) und durch das Trägerelement (2) verläuft.

5. Elektrisch betriebene Heizeinheit nach einem der vorhergehenden Ansprüche, **dadurch gekennzeichnet, dass** das Heizelement (10) entgegengesetzte erste und zweite ebene Hauptanlageflächen (11, 12) definiert, wobei die erste Anlagefläche (11) des Heizelements (10) dazu konfiguriert ist, an dem Kontaktelement (14) anzuliegen, und die zweite Anlagefläche (12) des Heizelements (10) dazu konfiguriert ist, an dem Wärmeübertragungselement (15) anzuliegen, wobei das Wärmeübertragungselement (15) einen Kontaktbereich (21) umfasst, der eine ebene Kontaktfläche (21) zum in Eingriff kommen mit der zweiten Anlagefläche (12) des Heizelements (10) in elektrischem und wärmeleitendem Kontakt festlegt.

6. Elektrisch betriebene Heizeinheit nach Anspruch 1, **dadurch gekennzeichnet, dass** das Wärmeübertragungselement (15) einen äußeren Umfangsabschnitt (22) aufweist, der um dessen Kontaktfläche (21) herum verläuft, und dass die zweite Verbindungseinrichtung (55) sich von dem äußeren Umfangsabschnitt (22) des Wärmeübertragungselements (15) aus erstreckt.

7. Elektrisch betriebene Heizeinheit nach einem der vorhergehenden Ansprüche, **dadurch gekennzeichnet, dass** die zweite Verbindungseinrichtung (55) ein zweites Verbindungselement (55, 56) aufweist, das an dem Wärmeübertragungselement (15) befestigt ist, wobei das zweite Verbindungselement eine Drahtaufnahmebohrung (57) angrenzend an ein distales Ende (59) desselben definiert, um einen zweiten elektrisch leitenden Draht (61) eines zweiten Pols der elektrischen Stromversorgung aufzunehmen, wobei das distale Ende (59) des zweiten Verbindungselements (55, 56) entweder zum Löten oder zum Crimpen des distalen Endes (59) des Verbindungselements (55, 56) an den zweiten elektrisch leitenden Draht ausgeführt ist.

8. Elektrisch betriebene Heizeinheit nach einem der vorhergehenden Ansprüche, **dadurch gekennzeichnet, dass** das Trägerelement (2) ein Gehäuse (5) umfasst, das einen hohlen Innenbereich (9) zur Aufnahme des Heizelements (10) darin festlegt, wobei das Gehäuse (5) eine Verbindungsöffnung (3) definiert, die mit dem hohlen Innenbereich (9) in Verbindung steht, und ein Plattenbauteil (4) umfasst, das sich neben der Verbindungsöffnung (3) um das Gehäuse (5) herum erstreckt, und dass das Wärmeübertragungselement (15) an einer Außenfläche (23) entweder des Gehäuses (5) oder des Plattenbauteils (4) anliegend neben der Verbindungsöffnung (3) angeordnet ist und die Verbindungsöffnung (3) verschließt.

9. Elektrisch betriebene Heizeinheit nach Anspruch 8, **dadurch gekennzeichnet, dass** die Befestigungseinrichtung (25, 27, 28) mindestens ein Paar miteinander in Eingriff bringbarer komplementärer Strukturen (25, 27, 28) umfasst, wobei eine der Strukturen (25) jedes Paares derselben auf dem Wärmeübertragungselement (15) vorgesehen ist und die andere des Paares der miteinander in Eingriff bringbaren komplementären Strukturen (25, 27, 28) auf dem Trägerelement (2) vorgesehen ist, wobei eine der miteinander in Eingriff bringbaren komplementären Strukturen (25, 27, 28) jedes Paares derselben ein Halteelement (25) umfasst, das sich von dem Trägerelement (2) oder dem Wärmeübertragungselement (15) aus erstreckt, und die andere des Paares der miteinander in Eingriff bringbaren komplementären Strukturen (25, 27, 28) jedes Paares derselben eine entsprechende Aufnahme (27, 28) umfasst, die in dem jeweils anderen des Trägerelements (2) oder des Wärmeübertragungselements (15) angeordnet ist.

10. Elektrisch betriebene Heizeinheit nach Anspruch 9, **dadurch gekennzeichnet, dass** jedes Halteelement (25) ein längliches Halteelement (25) umfasst, das sich von dem Trägerelement (2) oder dem Wärmeübertragungselement (15) aus erstreckt, und dass eine Eingriffsrippe (30) seitlich jedes Halteelements (25) verläuft, um in die zugehörige Aufnahme (27, 28) des jeweils anderen des Paares der miteinander in Eingriff bringbaren komplementären Strukturen (25, 27, 28) einzugreifen.

11. Elektrisch betriebene Heizeinheit nach Anspruch 10, **dadurch gekennzeichnet, dass** sich jedes Halteelement (25) von dem Wärmeübertragungselement (15) aus erstreckt, die Eingriffsrippe (30) jedes Halteelements (25) sich in einer allgemein auswärts gerichteten Richtung von dem Halteelement (25) aus erstreckt, und die Aufnahme (27, 28) jedes Paares der miteinander in Eingriff bringbaren komplementären Strukturen (25, 27, 28) einen Abschnitt (27) eines Randes (28) umfasst, der die Verbindungsöffnung (3) im Gehäuse (5) begrenzt.

12. Elektrisch betriebene Heizeinheit nach Anspruch 11, **dadurch gekennzeichnet, dass** die Eingriffsrippe (30) jedes Halteelements (25) dazu geformt ist, in einer Richtung durch die Verbindungsöffnung (3) zu passen, die durch den Rand (28) begrenzt ist, dessen entsprechender Teil die Aufnahme (27, 28) bildet, und mit dem Rand (28) in Eingriff zu geraten, wenn sie in die entgegengesetzte Richtung gedrückt wird.

13. Verfahren zur Herstellung einer elektrisch betriebenen Heizeinheit (1), wobei das Verfahren umfasst ein Anordnen eines PTC-Scheibenheizelements (10) zwischen einem elektrisch leitenden Wärmeübertragungselement (15) und einem elektrisch leitenden Kontaktelement (14), ein Befestigen des Heizelements (10) und des Kontaktelements (14) zwischen dem Wärmeübertragungselement (15) und einem Trägerelement (2), wobei das Heizelement (10) in elektrischem Kontakt mit dem Kontaktelement (14) und dem Wärmeübertragungselement (15) ist, ein Bereitstellen einer ersten Verbindungseinrichtung (38), die mit dem Kontaktelement (14) zusammenwirkt, um das Kontaktelement (14) mit einem ersten Pol einer elektrischen Stromversorgung elektrisch zu verbinden, und ein Bereitstellen einer zweiten Verbindungseinrichtung (55), die mit dem Wärmeübertragungselement (15) zusammenwirkt, um das Wärmeübertragungselement (15) mit einem zweiten Pol der elektrischen Stromversorgung zu verbinden, wobei das Verfahren ferner umfasst ein Drücken des Kontaktelements (14) und des Wärmeübertragungselements (15) in elektrischen Kontakt mit dem Heizelement (10) durch eine mit dem Trägerelement (2) und dem Kontaktelement (14) zusammenwirkende Druckfeder (34), die das Heizelement (10) sandwichartig zwischen dem Kontaktelement (14) und dem Wärmeübertragungselement (15) festhält, **dadurch gekennzeichnet, dass** die erste Verbindungseinrichtung (38) sich mittig vom Kontaktelement (14) erstreckend, mittig durch die Druckfeder (34) hindurch und mittig durch das Trägerelement (2) verlaufend angeordnet ist.

## Revendications

1. Une unité de chauffage à alimentation électrique comprenant un élément de support (2), un élément de transfert de chaleur électriquement conducteur (15), un élément de contact électriquement conducteur (14), un élément de chauffage en forme de disque à coefficient de température positif (10) situé entre l'élément de transfert de chaleur (15) et l'élément de contact (14), un moyen de fixation (25, 27, 28) fixant l'élément de chauffage (10) et l'élément de contact (14) entre l'élément de transfert de chaleur (15) et l'élément de support (2) et fixant l'élément de chauffage (10) en contact électrique avec l'élément de contact (14) et l'élément de transfert de chaleur (15), un premier moyen de connexion (38) coopérant avec l'élément de contact (14) pour connecter électriquement l'élément de contact (14) à un premier pôle d'une alimentation électrique, et un second moyen de connexion (55) coopérant avec l'élément de transfert de chaleur (15) pour connecter l'élément de transfert de chaleur (15) à un second pôle de l'alimentation électrique, dans lequel un moyen de poussée (34) comprenant un ressort de compression (34) pouvant coopérer avec l'élément de support (2) et l'élément de contact (14) pousse l'élément de contact (14) et l'élément de transfert de chaleur (15) en contact électrique avec l'élément de chauffage (10), et le moyen de fixation (25, 27, 28) est configuré pour fixer l'élément de chauffage (10) pris en sandwich de manière étanche entre l'élément de contact (14) et l'élément de transfert de chaleur (15), **caractérisée en ce que** le premier moyen de connexion (38) s'étend de manière centrale depuis l'élément de contact (14), de manière centrale à travers le ressort de compression (34), et de manière centrale à travers l'élément de support (2).

2. Une unité de chauffage à alimentation électrique selon la revendication 1, **caractérisée en ce que** le premier moyen de connexion (38) comprend une première attache de connexion (40) s'étendant depuis un bord périphérique (41) de l'élément de contact (14), l'attache de connexion (40) étant pliée au niveau d'un premier coude (42) adjacent au bord périphérique (41) de l'élément de contact (14) pour former une première partie (43) s'étendant vers l'intérieur depuis le bord périphérique (41), et sensiblement parallèle à l'élément de contact (14), l'attache de connexion (40) étant pliée au niveau d'un second pli (46) à un emplacement espacé du premier pli (42) et définissant une partie (45) s'étendant à partir de l'élément de contact (14).

3. Une unité de chauffage à alimentation électrique selon la revendication 2, **caractérisée en ce que** la partie (45) de l'attache de connexion (40) s'étendant depuis le deuxième coude (46) et depuis l'élément de contact (14) est pliée au niveau d'un troisième coude (49) définissant une partie intermédiaire (47) s'étendant entre les deuxième et troisième coudes (46, 49) et une partie distale (48) s'étendant à partir du troisième coude (49), la partie distale (48) étant pliée selon un angle d'environ 180° adjacent au troisième coude (49) pour se trouver sensiblement parallèle à la partie intermédiaire (47) de l'attache de connexion (40), la partie distale (48) de l'attache de connexion (40) s'étendant depuis le troisième coude (49) vers l'élément de contact (14), la partie distale (48) et la partie intermédiaire (47) de l'attache de connexion (40) sont espacées l'une de l'autre d'une partie adjacente au troisième coude (49) pour définir une ouverture (50) destinée à recevoir un premier fil électriquement conducteur (39) pour connecter l'élément de contact (14) à un premier pôle de l'alimentation électrique.

4. Une unité de chauffage à alimentation électrique selon l'une quelconque des revendications précédentes, **caractérisée en ce que** le second moyen de connexion (55) est situé sur l'élément de transfert de chaleur (15) à distance de l'élément de chauffage (10) et de l'élément de contact (14) et sur un côté de celui-ci, et s'étend parallèlement et à distance du premier moyen de connexion (38), et à travers l'élément de support (27).

5. Une unité de chauffage à alimentation électrique selon l'une quelconque des revendications précédentes, **caractérisée en ce que** l'élément de chauffage (10) définit des première et seconde surfaces de butée principales planes opposées (11, 12), la première surface de butée (11) de l'élément de chauffage (10) étant configurée pour venir en butée contre l'élément de contact (14), et la seconde surface de butée (12) de l'élément de chauffage (10) étant configurée pour venir en butée contre l'élément de transfert de chaleur (15), l'élément de transfert de chaleur (15) comprenant une zone de contact (21) définissant une surface de contact plane (21) destinée à s'engager avec la seconde surface de butée (12) de l'élément de chauffage (10) avec un engagement conducteur électrique et thermique.

6. Une unité de chauffage à alimentation électrique selon la revendication 5, **caractérisée en ce que** l'élément de transfert de chaleur (15) comprend une partie périphérique extérieure (22) s'étendant autour de sa zone de contact (21), et le second moyen de connexion (55) s'étend depuis la partie périphérique extérieure (22) de l'élément de transfert de chaleur (15).

7. Une unité de chauffage à alimentation électrique selon l'une quelconque des revendications précédentes, **caractérisée en ce que** le second moyen de connexion (55) comprend un second élément de connexion (55, 56) fixé à l'élément de transfert de chaleur (15), le second élément de connexion définissant un alésage de logement de fil (57) adjacent à une extrémité distale (59) de celui-ci pour loger un second fil électriquement conducteur (61) d'un second pôle de l'alimentation électrique, l'extrémité distale (59) du second élément de connexion (55, 56) étant configurée pour une soudure ou un sertissage de l'extrémité distale (59) de l'élément de connexion (55, 56) au second fil électriquement conducteur.

8. Une unité de chauffage à alimentation électrique selon l'une quelconque des revendications précédentes, **caractérisée en ce que** l'élément de support (2) comprend un logement (5) définissant une région intérieure creuse (9) pour y loger l'élément de chauffage (10), le logement (5) définissant une ouverture de communication (3) communiquant avec la région intérieure creuse (9), et comprenant un élément de plaque (4) s'étendant autour du logement (5) adjacent à l'ouverture de communication (3), et l'élément de transfert de chaleur (15) est situé en butée contre une surface extérieure (23) de l'un du logement (5) et de l'élément de plaque (4) adjacent à l'ouverture de communication (3) et fermant l'ouverture de communication (3).

9. Une unité de chauffage à alimentation électrique selon la revendication 8, **caractérisée en ce que** les moyens de fixation (25, 27, 28) comprennent au moins une paire de formations complémentaires inter-engageables (25, 27, 28), l'une des formations (25) de chaque paire étant prévue sur l'élément de transfert de chaleur (15) et l'autre de la paire de formations complémentaires inter-engageables (25, 27, 28) étant prévue sur l'élément de support (2), l'une des formations complémentaires inter-engageables (25, 27, 28) de chaque paire comprenant un élément de retenue (25) s'étendant depuis l'un de l'élément de support (2) et de l'élément de transfert de chaleur (15), et l'autre de la paire de formations complémentaires inter-engageables (25, 27, 28) de chaque paire comprenant un récepteur correspondant (27, 28) situé dans l'autre de l'élément de support (2) et de l'élément de transfert de chaleur (15).

10. Une unité de chauffage à alimentation électrique selon la revendication 9, **caractérisée en ce que** chaque élément de retenue (25) comprend un élément de retenue allongé (25) s'étendant à partir de l'élément de support (2) ou de l'élément de transfert de chaleur (15), et une arête d'engagement (30) s'étend latéralement à partir de chaque élément de retenue (25) pour engager le récepteur correspondant (27, 28) de l'autre de la paire de formations complémentaires inter-engageables (25, 27, 28).

11. Une unité de chauffage à alimentation électrique selon la revendication 10, **caractérisée en ce que** chaque élément de retenue (25) s'étend à partir de l'élément de transfert de chaleur (15), l'arête d'engagement (30) de chaque élément de retenue (25) s'étend dans une direction généralement vers l'extérieur à partir de l'élément de retenue (25), et le récepteur (27, 28) de chaque paire de formations complémentaires pouvant s'engager l'une dans l'autre (25, 27, 28) comprend une partie (27) d'un rebord (28) définissant l'ouverture de communication (3) dans le logement (5).

12. Une unité de chauffage à alimentation électrique selon la revendication 11, **caractérisée en ce que** l'arête d'engagement (30) de chaque élément de retenue (25) est formée pour passer dans une direction à travers l'ouverture de communication (3) définie par le rebord (28), dont la partie correspondante forme le récepteur (27, 28), et pour engager le rebord (28) lorsqu'il est poussé dans la direction opposée.

13. Un procédé de production d'une unité de chauffage (1) à alimentation électrique, le procédé comprenant le positionnement d'un élément de chauffage en forme de disque à coefficient de température positif (10) entre un élément de transfert de chaleur électriquement conducteur (15) et un élément de contact électriquement conducteur (14), fixer l'élément de chauffage (10) et l'élément de contact (14) entre l'élément de transfert de chaleur (15) et un élément de support (2), l'élément de chauffage (10) étant en contact électrique avec l'élément de contact (14) et l'élément de transfert de chaleur (15), fournir un premier moyen de connexion (38) coopérant avec l'élément de contact (14) pour connecter électriquement l'élément de contact (14) à un premier pôle d'une alimentation électrique, et la fourniture d'un second moyen de connexion (55) coopérant avec l'élément de transfert de chaleur (15) pour connecter l'élément de transfert de chaleur (15) à un second pôle de l'alimentation électrique, dans lequel le procédé comprend en outre le fait de pousser l'élément de contact (14) et l'élément de transfert de chaleur (15) en contact électrique avec l'élément de chauffage (10) par un ressort de compression (34) pouvant coopérer avec l'élément de support (2) et l'élément de contact (14), et fixer l'élément de chauffage (10) pris en sandwich de manière étanche entre l'élément de contact (14) et l'élément de transfert de chaleur (15), **caractérisé en ce que** le premier élément de connexion (38) est prévu pour s'étendre de manière centrale depuis l'élément de contact (14), de manière centrale à travers le ressort de compression (34), et de manière centrale à travers l'élément de support (2).
